**Europäisches Patentamt**

**European Patent Office**

**Office européen des brevets**

(11) Veröffentlichungsnummer: **0 386 410**
A1

## (12) EUROPÄISCHE PATENTANMELDUNG

(21) Anmeldenummer: **90100724.5**

(22) Anmeldetag: **15.01.90**

(51) Int. Cl.⁵: **F28D 20/00, A47J 36/28, A61F 7/00, C01B 3/00**

Ein Antrag gemäss Regel 88 EPÜ auf Berichtigung der Ansprüche 1-5 liegt vor. Über diesen Antrag wird im Laufe des Verfahrens vor der Prüfungsabteilung eine Entscheidung getroffen werden (Richtlinien für die Prüfung im EPA, A-V, 2.2).

(30) Priorität: **04.03.89 DE 3907084**

(43) Veröffentlichungstag der Anmeldung:
**12.09.90 Patentblatt 90/37**

(84) Benannte Vertragsstaaten:
**CH ES FR GB IT LI SE**

(71) Anmelder: **BATTELLE-INSTITUT E.V.**
**Am Römerhof 35 Postfach 90 01 60**
**D-6000 Frankfurt am Main 90(DE)**

(72) Erfinder: **Marinescu-Pasoi, Lucian, Dr.**
**Hansaallee 110**
**D-6000 Frankfurt am Main(DE)**
Erfinder: **Behrens Ulrich**
**Chattenweg 14**
**D-6368 Bad Vilbel(DE)**
Erfinder: **Langer Günter**
**Am Kirschenberg 2**
**D-6365 Rosbach 1(DE)**

(54) **Reversibler Speicher für Medien sowie Anwendung des Speichers.**

(57) Beschrieben wird ein reversibler Speicher für Medien, der aus einem Speicherkern (1), einem Zwischenspeicher (2), einem Katalysator (3) und einer Schutzhülle (4) besteht. Beim Öffnen der Schutzhülle erwärmt sich das gespeicherte Medium in der Katalysatorschicht, bedingt durch den dann erfolgenden Sauerstoffzutritt. Der Speicher kommt ohne eine äußere Wärmequelle aus, weil die Bindungsenergie im Zwischenpeicher viel kleiner ist als diejenige des Hauptspeichers, so daß der Zwischenspeicher als Starter für die Reaktion dient.

Anwendungen des erfindungsgemäßen Speichers werden insbesondere zum Erwärmen von Lebensmitteln erschließlich Getränken gesehen, für die Wärmebehandlung von unter Schock stehenden Patienten und als schnell ansprechender Wasserstoffspeicher zur Abgabe von Wasserstoff ohne die Zufuhr von Fremdenergie.

Fig.1

EP 0 386 410 A1

### Reversibler Speicher für Medien sowie Anwendung des Speichers

Die Erfindung bezieht sich auf einen reversiblen Speicher für Medien mit einem Speicherkern, der bei Erwärmung das gespeicherte Medium abgibt bzw. an den das Medium unter Wärmeabgabe angelagert wird.

Derartige reversible Speicher sind in mehreren Ausführungsformen bekannt geworden, wobei insbesondere auf die Metallhydride Bezug genommen wird.

Ein Nachteil der bekannten Speicher ist es aber, daß sie zum Starten eine äußere Energiezufuhr benötigen. Diese steht aber nicht immer zur Verfügung.

Der Erfindung liegt daher die Aufgabe zugrunde, einen reversiblen Speicher für Medien vorzuschlagen, der ohne eine äußere Energiezufuhr das gespeicherte Medium abgibt.

Zur Lösung dieser Aufgabe ist die Erfindung dadurch gekennzeichnet, daß der Speicherkern an wenigstens einem Zwischenspeicher, dieser an jeweils einem Katalysator und dieser wiederum an einer luftdichten Schutzhülle anliegt, die den oder die Katalysatoren vor Luftzutritt schützt.

Entfernt man die Schutzhülle im Bereich des Katalysators, so tritt Luftsauerstoff hinzu und dieser bewirkt in der Katalysatorschicht die Verbrennung des dort über den Zwischenspeicher an den Katalysator abgegebenen Mediums, beispielsweise Wasserstoffs. Diese Erwärmung startet also den Vorgang, so daß jetzt das im Speicherkern gespeicherte Medium, bedingt durch die Wärmeentwicklung in der Katalysatorschicht, das Medium, vorzugsweise ebenfalls Wasserstoff, ebenfalls abgibt, bis der Speicher entleert ist. Er kann dann wieder aufgeladen werden. Der Vorgang beruht unter anderem darauf, daß die Bindungsenergie des Mediums (Wasserstoff) in der Zwischenschicht viel kleiner ist als im Speicherkern, so daß das betreffende Medium auch bei Zimmertemperatur aus der als Starter dienenden Zwischenschicht in die Katalysatorschicht austreten kann und dort bei Luftzufuhr ausreichend Wärme abgibt, um den Prozeß auch im Hauptspeicher in Gang zu setzen und aufrecht zu erhalten.

Bevorzugte Materialien für den Speicherkern und den Zwischenspeicher sind Metallhydride. Es ist aber denkbar, daß der erfindungsgemäße Speicher auch bei anders ausgestalteten Speicherkernen und Zwischenspeichern arbeitet.

Eine bevorzugte Anwendung des erfindungsgemäßen Speichers besteht in der Erwärmung von Lebensmitteln (Speisen und Getränken). Hierzu ist es zwar bekannt, in den Zwischenraum einer doppelwandigen Dose gebrannten Kalk und Wasser einzugeben, so daß durch Luftzutritt beim Öffnen der Dose durch die dann einsetzende Reaktion Wärme entsteht, der die betreffende Speise erwärmt. Hierbei entstehen jedoch ätzende und toxische Stoffe, nämlich Löschkalk, verbunden mit der Gefahr, daß diese Stoffe in die zu erwärmende Speise eindringen, zumindest nach einer längeren Lagerungszeit der Dose. Auch ist eine Wiederverwendung praktisch ausgeschlossen und ein weiterer Nachteil ist das höhere Gewicht dieser bekannten Vorrichtung.

Eine weitere Anwendung des erfindungsgemäßen Speichers wird in der Wärmebehandlung von Patienten gesehen, insbesondere für Schockpatienten an einem Unfallort, wo eine entsprechend beheizte Decke nicht ohne weiteres zur Verfügung steht. Der Speicher ist hier als Foliendecke ausgebildet.

Außerdem wird eine wichtige Anwendung der Erfindung darin gesehen, diesen als schnell ansprechenden Wasserstoffspeicher zur Abgabe von Wasserstoff ohne Zufuhr von Fremdenergie auszubilden.

Wenn man für die Speicherung von Wasserstoff einen Metallhydridspeicher vorsieht, der von einem metallischen Katalysator umgeben ist, benutzt man die katalytische Verbrennung von Wasserstoff zur Wärmeentwicklung. Derartige metallische Katalysatoren sind an sich bekannt.

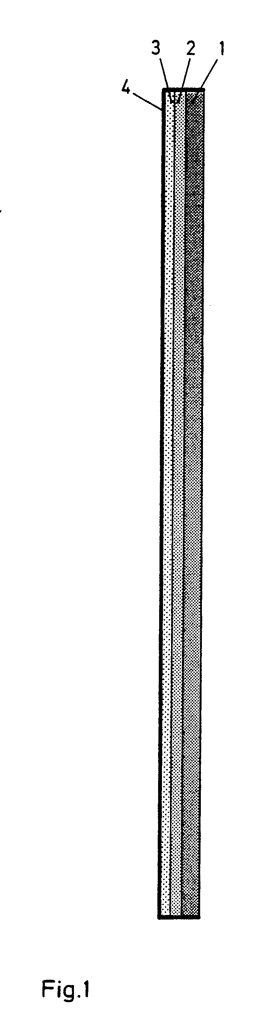

Die Erfindung wird im folgenden anhand eines Ausführungsbeispieles näher erläutert, aus dem sich weitere wichtige Merkmale ergeben. Die Figur zeigt schematisch den grundsätzlichen Aufbau eines erfindungsgemäßen Speichers.

Dieser besteht - in der Figur von rechts nach links - aus einem Speicherkern 1, der an einem Zwischenspeicher 1 anliegt. Dieser wiederum liegt an einem Katalysator 3 an, dessen Außenseite von einer Schutzhülle 4 abgeschlossen ist. Die Schutzhülle umgibt zumindest die Außenseite des Katalysators, so daß sichergestellt wird, daß keine Luft (Sauerstoff) Zutritt zum Katalysator hat. Es sei erwähnt, daß zur Seite des Zwischenspeichers hin dieser bzw. vorzugsweise der Speicherkern den Katalysator im allgemeinen ausreichend vor einer Luftzufuhr schützt.

Die Arbeitsweise des beschriebenen Speichers ist wie folgt.

Die Speicher 1,2 werden mit Wasserstoff beladen und gegen Sauerstoffzufuhr abgeschlossen.

Nach dem Öffnen der Schutzhülle 4 tritt der in dem Zwischenspeicher 2 eingelagerte Wasserstoff durch die äußere katalytische Schicht 3 aus. Er reagiert an der Oberfläche der katalytischen Schicht 3 mit dem in der Luft vorhandenen Sauerstoff. Bei dieser chemischen Reaktion wird Energie in Form von Wärme freigesetzt. Ein Teil dieser Wärme heizt die Vorrichtung auf, und weiterer Sauerstoff aus dem inneren Hydridspeicher 1 tritt nach außen und reagiert ebenfalls an der Oberfläche der katalytischen Schicht 3 mit dem in der Luft vorhandenen Sauerstoff. Die dabei freiwerdende Wärme wird dann zur Erwärmung eines anderen Mediums genutzt.

Eine Wiederverwendung ist möglich und aufgrund der relativ hohen Kosten für die Materialien der Vorrichtung auch anzustreben.

Es gibt mehrere Anwendungsgebiete für diese Vorrichtung.

- Erwärmung von Getränken und Speisen mit Hilfe einer selbsterwärmenden Vorrichtung. Hierbei sind drei Ausführungsbeispiele zu nennen.

1. Selbsterwärmendes Behältnis (Dose) mit Doppelwandung, das das zu erwärmende Medium enthält, und deren innere Wandungen nach dem oben genannten Prinzip aufgebaut sind. Nach dem Öffnen des Behältnisses dringt Luft zwischen die beiden Wandungen ein und Wärme wird freigesetzt.

2. Körper (Kugel, Zylinder etc.), der nach dem oben genannten Prinzip aufgebaut ist und der nach dem Entfernen der Schutzschicht in das zu erwärmende Medium eingetaucht wird.

3. Kocher, auf dem das zu erwärmende Medium in einem Behältnis erwärmt wird. Der Kocher besteht aus einem luftdichten Gefäß, das Körper (Kugeln, Zylindert etc.) enthält, die nach dem oben genannten Prinzip aufgebaut sind.Die Wärmeentwicklung wird durch gezielte Luftzufuhr geregelt. - Einsatz als schnell ansprechender Wasserstoffspeicher ohne Zufuhr von Fremdenergie:
Der Wasserstoffspeicher bestehend aus einem Behältnis, in dem sich Metallhydrid und Katalysator befinden, die nach dem oben genannten Prinzip aufgebaut sind. Durch kontrollierte Luftzufuhr wird ein Teil des gespeicherten Wasserstoffs umgesetzt und eine Erwärmung des Speichers und damit die Freisetzung des benötigten Wasserstoffs bewirkt. Die Wärmeentwicklung geschieht an dem Ort, an dem sich auch der gespeicherte Wasserstoff befindet. Damit ist eine sofortige und schnelle Freisetzung des Wasserstoffs gewährleistet. Zur Freisetzung des Wasserstoffs wird keine externe Wärmequelle benötigt.
In Kombination dieses Verfahrens mit einer Wärmerückführung aus Abgasen bei einem mit Wasserstoff betriebenen Kraftfahrzeug, können Hochtemperatur-Hydridspeicher genutzt werden. Diese Speicher haben eine höhere Wasserstoff-Speicherkapazität. Eine Nutzung von Hochtemperatur-Hydridspeichern war bisher ohne zusätzliche externe Wärmezufuhr nicht möglich, da die Wärmerückführung aus Verbrennungsprozessen im Motor für das Freisetzen des Wasserstoffs nicht ausreichte.
- Als Foliendecke in der Unfallmedizin zur Wärmebehandlung von Schockpatienten am Unfallort:
Die Körpertemperatur von Unfallopfern, die z.B. unter Schock stehen, darf nicht absinken. Nach dem oben genannten Prinzip können auch Folien aufgebaut sein, die auf einem wärmeisolierenden Träger aufgebracht sind und dann neben der Wärmeisolierung zusätzlich eine Wärmequelle darstellen. Die Folien sind zusammengelegt in einem luftdichten Behälter eingepackt, der am Unfallort geöffnet wird.

Die Vorteile des erfindungsgemäßen Speichers liegen insbesondere darin, daß eine Erwärmung von Medien in einem exergetisch günstigen niedrigen Temperaturbereich (z.B. <100° C) erfolgen kann. Die Vorrichtung benötigt keine äußere Energiezufuhr; die Wärmeabgabe erfolgt selbstregelnd bei geeigneter Wahl der konstruktiven Merkmale.

Die äußere katalytische Schicht hat darüber hinaus den Vorteil, daß sie eine Oxidation des Metallhydridspeichers verhindert. Hierdurch erst ist es möglich, diese Vorrichtung auch bei Vorhandensein von Sauerstoff zu betreiben.

Die Vorrichtung ist für einen Inselbetrieb und mobile Einsatzzwecke geeignet. Sie ist leicht zu bedienen und stellt im Vergleich zu Wasserstoff-Druckspeicher oder Flüssigwasserstoff-Speicher keine potentielle Gefahrenquelle für die Umgebung dar. Darüber hinaus ist sie umweltfreundlich, da es sich hierbei um eine wiederverwendbare Vorrichtung handelt und bei der Verbrennung des Wasserstoffs wegen der niedrigen Reaktionstemperatur als Abgas nur $H_2O$ entsteht.

Eine solche Anordnung von unterschiedlichen Schichten, wie sie in dieser Vorrichtung verwirklicht ist, kann auch für die Trennung von Wasser in Sauerstoff genutzt werden. Der oben beschriebene chemische Vorgang läuft dabei in der entgegengesetzten Richtung ab und der entstehende Sauerstoff wird im Speicher gebunden.

Kern der Erfindung ist also ein zur Speicherung von Medien, die bei einer chemischen Reaktion Energie abgeben und die zusätzlich mit einem oder mehreren Medienspeichern in Kontakt stehen, die auch als Zwischenspeicher genutzt werden können und mit einer Schicht in Kontakt stehen, die als Katalysator für die chemische Reaktion des gespeicherten Mediums dient und die ein Teil der Reaktionswärme direkt zur Erwärmung des oder der Speicher nutzt und damit eine Freisetzung des in diesen Speicher gespeicherten Mediums bewirkt. Die gesamte Vorrichtung ist durch geeignete Maßnahmen (Schutzhülle, luftdichtes Behältnis etc.) gegen die unkontrollierte Zufuhr des oder der Reaktionspartner des gespeicherten Mediums abgeschlossen.

Der Aufbau der Vorrichtung kann schalenförmig, z.b. als Kugel, Zylinder etc., oder flächenförmig, z.B. als Folie etc. sein.

## Ansprüche

1. Reversibler Speicher für Medien mit einem Speicherkern, der bei Erwärmung das gespeicherte Medium abgibt bzw. an den das Medium unter Wärmeabgabe angelagert wird,
**dadurch gekennzeichnet,**
daß der Speicherkern (1) an wenigstens einem Zwischenspeicher (2), dieser an jeweils einem Katalysator (3) und dieser wiederum an einer luftdichten Schutzhülle (4) anliegt, die den oder die Katalysatoren (3) vor Luftzutritt schützt.

2. Speicher nach Anspruch 1,
**dadurch gekennzeichnet,**
daß der Speicherkern (1) und der Zwischenspeicher (2) Metallhydride sind.

3. Anwendung des Speichers nach Anspruch 1 oder 2 zum Erwärmen von Lebensmitteln.

4. Anwendung des als Foliendecke ausgebildeten Speichers nach Anspruch 1 oder 2 zur Wärmebehandlung von Patienten.

5. Anwendung des Speichers nach Anspruch 1 oder 2 als schnell ansprechender Wasserstoffspeicher zur Abgabe von Wasserstoff ohne Zufuhr von Fremdenergie.

Fig.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | GB-A-2 051 342 (DAIMLER-BENZ AG) * Seite 1, Zeilen 34-63 * --- | 1,2 | F 28 D 20/00 A 47 J 36/28 A 61 F 7/00 C 01 B 3/00 |
| A | DE-A-2 800 903 (SIEMENS AG) * Seite 10, Zeilen 7-26; Figur 1 * --- | 1,2 | |
| A | US-A-3 993 577 (BLACK et al.) * Spalte 2, Zeilen 18-26; Spalte 11, Zeile 42 - Spalte 12, Zeile 2; Figur 4 * --- | 1,2,4,5 | |
| A | EP-A-0 130 757 (THE STATE OF ISRAEL MINISTRY OF DEFENCE MILITARY INDUSTRIES) * Seite 5, Zeile 28 - Seite 7, Zeile 21; Figur 1 * --- | 1,3 | |
| A | JOURNAL OF THE LESS-COMMON METALS, Band 106, 1985, Seiten 263-267, Elsevier Sequoia, NL; Y.K. VIJAY: "New design for a hydrogen storage system" ----- | 1,2,5 | RECHERCHIERTE SACHGEBIETE (Int. Cl.5) F 24 J C 01 B F 28 D A 61 F A 47 J |

Der vorliegende Recherchenbericht wurde für alle Patentansprüche erstellt

| Recherchenort | Abschlußdatum der Recherche | Prüfer |
|---|---|---|
| DEN HAAG | 22-05-1990 | BELTZUNG F.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)